# EUROPEAN PATENT SPECIFICATION

(11) **EP 3 482 741 B1**
(45) Date of publication and mention of the grant of the patent: **15.07.2020**
(21) Application number: 17200556.3
(22) Date of filing: 08.11.2017
(51) Int. Cl.: A61K 8/26, A61K 8/44, A61Q 15/00, A61Q 17/00, A61K 8/19, A01N 59/06

(54) **ANTIMICROBIAL ANTIPERSPIRANT COMPOSITIONS**
ANTIMIKROBIELLE SCHWEISSHEMMENDE ZUSAMMENSETZUNGEN
COMPOSITIONS ANTIPERSPIRANTES ANTIMICROBIENNES

(43) Date of publication of application: 15.05.2019
(73) Proprietor: Unilever PLC, London Greater London EC4Y 0DY (GB); Unilever N.V., 3013 AL Rotterdam (NL)
(72) Inventor: Cornmell, Robert Joseph, Wirral, Merseyside, CH63 3JW (GB); James, Alexander Gordon, Bedfordshire, MK44 1LQ (GB); Luckwell, Craig James, Leeds, West Yorkshire LS14 2AR (GB); Waterfield, Philip Christopher, Wirral, Merseyside CH63 3JW (GB)
(74) Representative: Whaley, Christopher

(56) References cited:
- WO-A1-2016/066528
- WO-A1-2016/078991
- WO-A2-2012/021356

## Description

### Field of Invention

The present invention is concerned with antiperspirant compositions and their use to treat the surface of the human body. The invention is particularly concerned with the use of antiperspirant compositions as antimicrobial agents.

### Background

Antimicrobial compositions and their use to treat the surface of the human body has been known for many decades. Such use has been primarily to reduce perspiration, although the commensurate benefit of reducing body odour is also known.

Antiperspirant compositions comprising aluminium sesquichlorohydrate (ASCH) are also known, as are methods of activating such salts using water-soluble calcium salts and amino acids such as glycine.

WO 14/187685 (Unilever) discloses antiperspirant compositions comprising ASCH, water-soluble calcium salt and amino acid and methods for their manufacture.

WO 14/187802 (Unilever) additionally discloses that antiperspirant compositions comprising ASCH, water-soluble calcium salt and amino acid may be used to deliver excellent antiperspirancy and reduced staining as well as excellent antiperspirancy and reduced stickiness.

WO 17/076836 (Unilever) discloses that antiperspirant compositions comprising ASCH, water-soluble calcium salt and amino acid, when packaged in particular aerosol dispensers, can provide a more environmentally sustainable aerosol product.

### Summary of Invention

It is an object of the present invention to deliver a superior antimicrobial benefit to the surface of the human body. This is achieved by the topical delivery of specific antiperspirant active systems and compositions known for their superior antiperspirancy efficacy, but not previously known for their superior antimicrobial performance.

The invention has particular relevance to microbial species present on the surface of the human by, especially those species associated with the development of body malodour.

Aspects of the invention are defined in independent claims 1 and 12.

### Detailed Description

The present invention concerns the antimicrobial benefit delivered on topical application of certain antiperspirant actives and/or compositions comprising them. We have found that AASCH is particularly effective in this regard.

The present invention has particular benefit for the topical treatment of the more malodorous regions of the human body, in particular the underarm regions or axillae.

Herein, "antimicrobial benefit" should be understood to refer to a reduction in Staphylococci present on the surface of the human body. A reduction in staphylococci bacterial numbers is a particular benefit of the present invention and is associated with a reduction in malodour.

Herein, "activated" means increased in antiperspirancy efficacy.

Herein, "comprising" means "including" but not necessarily "consisting of" or "composed of." In other words, the listed steps or options need not be exhaustive.

Herein, all percentages, amounts and ratios are by weight unless otherwise indicated.

Herein, numerical ranges expressed in the format "from x to y" are understood to include x and y. When for a specific feature multiple preferred ranges are described in the format "from x to y", it is understood that all ranges combining the different endpoints are also contemplated.

The AASCH used in the present invention has aluminium to chloride molar ratio of from 1.25:1 to 1.82:1 and preferably 1.54:1 to 1.82:1. It is "activated" by use of a water-soluble calcium salt and an amino acid.

In order for the antiperspirant to become activated, it is important to have sufficient calcium present relative to the amount of aluminium present. The molar ratio of calcium to aluminium is typically at least 1:40, preferably at least 1:30, more preferably at least 1:20 and most preferably at least 1:15. It is not advantageous to have the calcium concentration in excess of the aluminium concentration, indeed it is preferred that the calcium concentration is no more than half that of the aluminium concentration and more preferred that it is no more than a fifth of said concentration. For the preferred molar ratios of calcium to aluminium, it is independently preferred that this ratio is no greater than 1:2 and more preferred that it is no greater than 1:5.

In particularly preferred embodiments, the molar ratio of calcium to aluminium is at least 1:15 and preferably no greater than 1:5.

A preferred water-soluble calcium salt for use in the present invention is calcium chloride.

Herein, references to molar amounts and ratios of "aluminium" are calculated on the basis of mono-nuclear aluminium, but include aluminium present in poly-nuclear species; indeed, most of the aluminium in the salts of relevance is present in poly-nuclear species.

In order for the antiperspirant to become activated, it is important to have sufficient amino acid present relative to the amount of aluminium present. The molar ratio of amino acid to aluminium is preferably at least 1:20, more preferably at least 1:15. It is not advantageous to have the amino acid concentration in excess of the aluminium concentration; hence, the molar amino acid to aluminium is preferably from 1:20 to 1:1, more preferably from 1:15 to 1:2 and most preferably from 1:10 to 1:2.

Preferred amino acids for use in the AASCH are glycine, alanine, valine and proline. A particularly preferred amino acid is glycine.

The presence of both calcium and amino acid in the AASCH is essential. In preferred embodiments, the molar ratio of calcium to aluminium is at least 1:40 and the molar ratio of amino acid to aluminium is at least 1:20. In further preferred embodiments the molar ratio of calcium to aluminium is at least 1:20 and the molar ratio of amino acid to aluminium is at least 1:10. In particularly preferred embodiments the molar ratio of calcium to aluminium is from 1:20 to 1:5 and the molar ratio of amino acid to aluminium is from 1:10 to 1:1.

In certain especially preferred embodiments, the molar ratio of calcium to aluminium is from 1:15 to 1:5 and the molar ratio of amino acid to aluminium is from 1:10 to 1:2.

AASCH as used in the present invention generally has a relatively high content of what is commonly termed Band III material, as determined by SEC (Size Exclusion Chromatography) analysis. The SEC technique employed is well known in the art and is described in further detail in US 4,359,456 (Gosling). The SEC band commonly referred to as Band III is designated as "Peak 4" in EP 1,104,282 B1 by Gillette.

Herein, "Band III content" refers to the integrated area in the Band III region of the SEC chromatograph relative to the total integrated area in all of the regions corresponding to aluminium species; that is to say, Bands I, II, III, and IV.

In particular embodiments of the invention, AASCH used in accordance with the present invention preferably has a Band III content of at least 30%, more preferably at least 50% and most preferably at least 60%.

AASCH as used in the present invention is typically produced by heating together an aqueous solution of ASCH, water-soluble calcium salt and amino acid. The mixture is generally heated to at least 65°C, preferably to at least 75°C, and more preferably to at least 85°C. The mixture is typically held at elevated temperature for at least two hours.

The aqueous solution produced by the activation process described above may be dried by techniques known in the art, notably spray drying, to give a dried antiperspirant salt. Such dried antiperspirant salts may be used in a variety of compositions, including aerosols, sticks and soft solids. It will be realised that such compositions may be essentially anhydrous, having less than 1% by weight of free water or may be anhydrous, having less than 0.1% by weight of free water.

Herein, "free water" excludes any water of hydration associated with the antiperspirant salt or other component added to a particular composition, but includes all other water present.

The AASCH used in the present invention may be formulated into a cosmetic composition. Such compositions are topically applied to deliver the use according to the invention.

The level of AASCH used in cosmetic compositions is typically from 5% to 50% by weight of the total composition excluding any volatile propellant that may be present. On the same basis, this level is preferably from 5 to 40%, more preferably from 5 to 30% by weight and most preferably from 10 to 25% by weight.

Cosmetic compositions used in accordance with the invention may comprise a range of additional components according to their format. Formats include roll-ons, aerosols, sticks and soft solids. Preferably, the AASCH is applied from a cosmetic roll-on or aerosol composition.

Preferred cosmetic roll-on compositions are aqueous compositions, in particular oil-in-water emulsion compositions. Such compositions are preferably stabilised by a non-ionic emulsifier or emulsifier system. Emulsifier systems that exist as a lamellar phase in the composition are particularly preferred.

Herein "aqueous compositions" are compositions having a continuous phase that is predominately water; that is to say, greater than 50% water.

Preferred aerosol compositions are anhydrous compositions comprising a volatile propellant.

A preferred additional component used alongside AASCH in the present invention is an oil.

Herein, the term "oil" signifies a water-insoluble organic material that is liquid at 20°C. Any material having a solubility of less than 0.1g/100g at 20°C is considered to be insoluble.

A preferred oil is a fragrance oil, sometimes alternatively called a perfume oil. The fragrance oil may comprise a single fragrance component or more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

The total amount of oil in the composition is preferably from 0.1 to 20%, more preferably from 0.5 to 10%, and most preferably at from 2 to 8% by weight of the total composition.

When an aqueous composition is employed, it is very suitable for dispensing via a roll-on dispenser. Commonly, the dispenser is wiped across the skin between 4 and 10 strokes. Commonly, from 0.2 to 0.5 g of the composition is deposited in each armpit per application.

When an aerosol composition is employed, it is typically applied from an aerosol device comprising a pressurised container and release system comprising a valve, release button and exit orifice producing a spray. Such dispensing is typically done at a range of from 10 cm to 20 cm from the skin and for from 1 to 4 seconds per application. Commonly, from 1 to 3 g of the composition is released per application, but it must be borne in mind that a large part of this is typically volatile propellant.

### Examples

### Example 1

The aerosol compositions detailed in Table 1 were prepared by methods known in the art.

**Table 1**

| Raw material | | Amounts (% by weight) | |
|---|---|---|---|
| INCI name | Trade name (supplier) | Example 1 | Comparative Example A |
| Cylcomethicone | DC245 | 8.4 | 8.4 |
| | (Dow Corning) | | |
| Disteardimonium | Bentone 38V | 0.5 | 0.5 |
| Hectorite | (Elementis) | | |
| Propylene carbonate | Propylene carbonate | 0.1 | 0.1 |
| | (Jeffesol) | | |
| AASCH* | Comprised of: | 1.00 | --- |
| | ASCH (anhydrous) | 0.590 } | |
| | CaCl₂ | 0.072 } | |
| | Glycine | 0.232 } | |
| | Water of hydration | 0.152 } | |
| Aluminium | AACH-3117 | --- | 2.00 |
| chlorohydrate | (Summit) | | |
| PPG-14 Butyl Ether | Fluid AP | --- | 2.0 |
| | (Dow) | | |
| Butane, isobutane, | CAP 40 | 90.0 | 87.0 |
| propane | (Calor) | | |

| | | | |
|---|---|---|---|
| * This raw material was prepared from ASCH (having an Al: Cl ratio of between 1.6 and 1.7), anhydrous calcium chloride and glycine in the weight ratio indicated in the table. This mixture was heated as a 30% aqueous solution to about 87°C and kept at this temperature for two hours. The solution was then spray-dried to give a particulate solid having a particle size distribution such that at least 80% (by volume) of the particles were from 10 to 75 microns. The molar ratio of Al: Ca: glycine in this sample was 10: 1.0: 4.65, respectively. | | | |

The samples detailed in Table 1 were tested using panels of 48 females. At the start of the test, panellists were washed with unfragranced soap and the different products (Example 1 or Comparative Example A) were applied to each axilla via a 2-second spray, applied approximately 6 inches from the underarm. Product application was randomised to take into account any left/right bias. The mean dosage achieved was 1.92 g for Example 1 and 1.95 g Comparative Example A.

Sampling was carried out at 24 hours and 48 hours after sample application. Each panellist provided a single 24 hour or 48 hour sample from both axillae on one of the test days using the scrub cup technique as described by Taylor et al. (International Journal of Cosmetic Science, Vol 25, 2003, pp 137-145).

Samples were diluted and dispensed onto agar plates containing culture media selective for staphylococci, corynebacteria and Gram-positive anaerobic cocci (GPAC), using an automatic spiral plater. After incubation, plates were enumerated using a colony counter and the data used to calculate log₁₀ transformed colony-forming units per square cm skin (Log CFU / cm²). Bacterial numbers were averaged across the relevant daily sample collections, and the data analysed statistically.

This test assessed the numbers of the main bacterial types resident in the axillae, namely staphylococci, corynebacteria and GPAC. The results arsing are shown in Table 2. "Bacterial numbers" referred to are Log₁₀ CF bacteria per sq. cm skin.

**Table 2**

| Example | Bacterial numbers after 24 hours | | |
|---|---|---|---|
| | *Staphylococcus* | *Corynebacterium* | GPAC |
| 1 | 4.26 | 2.65 | 2.62 |
| A | 4.89 | 3.13 | 3.71 |
| P value (1 vs. A): | 0.0189 | 0.2773 | 0.042 |

Example 1 gave a greater reduction in bacterial numbers that the control, despite having a much lower level of antiperspirant active present. The differences were significant at the 95% level for the *Staphylococcus* and GPAC species.

### Example 2

The aerosol samples detailed in Table 3 were prepared by methods known in the art. The AASCH used was the same as that used in Example 1 and the samples were tested by essentially the same method as used for Example 1, except that 50 female panellists were used for the 24-hour test and 49 were used for the 48-hour test. The results are shown in Table 4.

It was found that Example 2 gave a greater numeric reduction in bacterial numbers against each bacterial species after both 24 and 48 hours; however, the difference was only significant at the 95% level for the *Staphylococcus.*

**Table 3**

| Raw material | | Amounts (% by weight) | |
|---|---|---|---|
| INCI name | Trade name (supplier) | Example 2 | Comparative Example B |
| Cylcomethicone | DC245 | 7.4 | 7.4 |
| | (Dow Corning) | | |
| Disteardimonium Hectorite | Bentone 38V | 0.5 | 0.5 |
| | (Elementis) | | |
| Propylene carbonate | Propylene carbonate | 0.1 | 0.1 |
| | (Jeffesol) | | |
| AASCH | Comprised of: | 2.00 | --- |
| | ASCH (anhydrous) | 1.180} | |
| | CaCl₂ | 0.144} | |
| | Glycine | 0.464} | |
| | Water | 0.304} | |
| Aluminium chlorohydrate | AACH-3117 | --- | 2.00 |
| | (Summit) | | |
| Butane, isobutane, propane | CAP 40 | 90.0 | 90.0 |
| | (Calor) | | |

**Table 4**

| Example | Bacterial numbers after... | | | | | |
|---|---|---|---|---|---|---|
| | *Staphylococcus* | | *Corynebacterium* | | GPAC | |
| | 24 hrs. | 48 hrs. | 24 hrs. | 48 hrs. | 24 hrs. | 48 hrs. |
| 2 | 4.04 | 4.49 | 2.42 | 3.30 | 1.52 | 1.86 |
| B | 4.72 | 5.28 | 2.92 | 3.82 | 2.13 | 2.41 |
| P value (2 vs. B): | 0.0175 | 0.0211 | 0.3415 | 0.231 | 0.302 | 0.3495 |

### Example 3

The roll-on samples detailed in Table 5 were prepared by methods known in the art.

The "AASCH solution" was prepared from ASCH (having an Al: Cl ratio of between 1.6 and 1.7), anhydrous calcium chloride, glycine and water in the weight ratio indicated in the table. This mixture (33% total solids) was heated to about 87°C and kept at this temperature for two hours. After this time, the solution was allowed to cool and used directly in the preparation of Example 3. The molar ratio of Al: Ca: glycine in the AASCH was 10: 1.0: 1.16, respectively.

Microbiological tests were conducted by essentially the same method as used for Example 1, except that the target dosage was 0.30g (+/- 0.03g) and was applied using a conventional roll-on dispenser. The mean dosage achieved was 0.033g for both Example 3 and Comparative Example C. The panel comprised 48 female volunteers.

In addition to the microbiological testing, a malodour assessment was performed. Assessment was performed by at least three expert assessors 5 hours and 24 hours after application, scoring the malodour intensity on a scale of 0-5. The results are shown in Table 6.

**Table 5**

| Raw material | | Amounts (% by weight) | |
|---|---|---|---|
| INCI name | Trade name (supplier) | Example 3 | Comparative Example C |
| Aqua | DI water | 49.505 | 63.95 |
| AASCH solution | Comprised of: | | --- |
| | ASCH (anhydrous) | 12.00} | |
| | CaCl₂ | 1.50} | |
| | Glycine | 1.175} | |
| | Water | 29.77} | |
| Aluminium chlorohydrate Aqua | Chlorhydrol 50 (Summit) including water at: | --- | 12.00 (anhydrous) 18.00 |
| Steareth-20 | Brij 78 (Croda) | 0.90 | 0.90 |
| Steareth-2 | Brij 72P (Croda) | 2.30 | 2.30 |
| Silica dimethyl silylate | HDK H30 (Wacker) | 0.70 | 0.70 |
| Helianthus Annuus Seed Oil | Sunflower Seed Oil (AAK) | 2.00 | 2.00 |
| Disodium EDTA | EDTA (Sigma-Aldrich) | 0.10 | 0.10 |
| Pentaerythrityl tetra-di-t-butyl hydroxyhydrocinnamate | Tinogard TT (BASF) | 0.05 | 0.05 |

**Table 6**

| Example | Mean malodour score | |
|---|---|---|
| | After 5hrs. | After 24 hrs. |
| 3 | 1.94 | 2.26 |
| C | 1.98 | 2.33 |
| P value (3 vs. C): | 0.2755 | 0.0212 |

From Table 6, it can be seen that there was no significant difference between the malodour scores at 5 hours; however, after 24 hours, Example 3 gave a significantly lower malodour score at the 95% level of confidence.

The results of the antimicrobial tests revealed no significant differences in microbial numbers after 5 hours. For corynebacteria and GPAC, the same was also true after 24 hours; however, a detailed statistical analysis of the staphyloccal data across the full panel did show a significant difference at 24 hours - Example 3 resulting in lower bacterial numbers.

## Claims

1. Non-therapeutic use of an activated aluminium sesquichlorohydrate (AASCH) for reducing malodour caused by Staphylococci on the surface of human body, **characterised in that** the AASCH comprises a water-soluble calcium salt, an amino acid and aluminium sesquichiorohydrate (ASCH) of formula Al₂OH_{4.4}Cl_{1.6} to Al₂OH_{4.9}Cl_{1.1}.

2. Non-therapeutic use of an activated aluminium sesquichiorohydrate (AASCH) according to claim 1, **characterised in that** the AASCH comprises a water-soluble calcium salt, an amino acid and aluminium sesquichiorohydrate (ASCH) of formula Al₂OH_{4.7}Cl_{1.3} to Al₂OH_{4.9}Cl_{1.1}.

3. Use according to claim 1 or claim 2, wherein the molar ratio of basic aluminium salt to calcium is from 1:20 to 1:5 and the molar ratio of basic aluminium salt to amino acid is from 1:10 to 1:1.

4. Use according to any of the preceding claims, wherein the amino acid is glycine and the water-soluble calcium salt is calcium chloride.

5. Use according to any of the preceding claims, wherein the basic aluminium chloride has a Band III content measured by SEC of greater than 30%.

6. Use according to any of the preceding claims, wherein the AASCH is applied from a cosmetic composition selected from aerosol, liquid, soft solid or solid, preferably from aerosol or liquid.

7. Use according to claim 5, wherein the AASCH comprises from 5 to 50% by weight of total composition excluding any volatile propellant present.

8. Use according to any of the preceding claims, wherein the AASCH is manufactured by a process comprising the steps of: i) mixing ASCH, water-soluble calcium salt, amino acid and water, ii) heating the mixture to a temperature of at least 65°C, and iii) cooling the mixture to ambient temperature.

9. Use according to any of the preceding claims, wherein the antimicrobial benefit lasts for at least 5 hours, preferably at least 24 hours.

10. Use according to any of the preceding claims, wherein the AASCH is topically applied to the underarm regions of human body.

11. Use according to any of the preceding claims, wherein the AASCH is applied from a cosmetic roll-on or aerosol composition.

12. Activated aluminium sesquichlorohydrate (AASCH) for use as an antimicrobial against Staphlococci on the surface of human body, **characterised in that** the AASCH comprises a water-soluble calcium salt, an amino acid and ASCH of formula Al₂OH_{4.4}Cl_{1.6} to Al₂OH_{4.9}Cl_{1.1}.

13. Activated aluminium sesquichlorohydrate (AASCH) for use as an antimicrobial according to claim 12, **characterised in that** the AASCH comprises a water-soluble calcium salt, an amino acid and ASCH of formula Al₂OH_{4.7}Cl_{1.3} to Al₂OH_{4.9}Cl_{1.1}.

## Patentansprüche

1. Nichttherapeutische Verwendung von einem aktivierten Aluminiumsesquichlorhydrat (AASCH) zur Reduzierung des schlechten Geruchs, der durch Staphylokokken auf der Oberfläche des menschlichen Körpers verursacht wird, **dadurch gekennzeichnet, dass** das AASCH ein wasserlösliches Calciumsalz, eine Aminosäure und Aluminiumsesquichlorhydrat (ASCH) der Formel Al₂OH_{4,4}Cl_{1,6} bis Al₂OH_{4,9}Cl_{1,1} umfasst.

2. Nichttherapeutische Verwendung von einem aktivierten Aluminiumsesquichlorhydrat (AASCH) nach Anspruch 1, **dadurch gekennzeichnet, dass** das AASCH ein wasserlösliches Calciumsalz, eine Aminosäure und Aluminiumsesquichlorhydrat (ASCH) der Formel Al₂OH_{4,7}Cl_{1,3} bis Al₂OH_{4,9}Cl_{1,1} umfasst.

3. Verwendung nach Anspruch 1 oder Anspruch 2, wobei das Molverhältnis von basischem Aluminiumsalz zu Calcium 1:20 bis 1:5 beträgt und das Molverhältnis von basischem Aluminiumsalz zu Aminosäure 1:10 bis 1:1 beträgt.

4. Verwendung nach irgendeinem der vorhergehenden Ansprüche, wobei die Aminosäure Glycin ist und das wasserlösliche Calciumsalz Calciumchlorid ist.

5. Verwendung nach irgendeinem der vorhergehenden Ansprüche, wobei das basische Aluminiumchlorid einen durch SEC gemessenen Band III-Gehalt von mehr als 30% aufweist.

6. Verwendung nach irgendeinem der vorhergehenden Ansprüche, wobei das AASCH aus einer kosmetischen Zusammensetzung angewendet wird, die aus Aerosol, Flüssigkeit, weichem Feststoff oder Feststoff, bevorzugt aus Aerosol oder Flüssigkeit, ausgewählt wird.

7. Verwendung nach Anspruch 5, wobei das AASCH 5 bis 50 Gew.-% der Gesamtzusammensetzung, ausgenommen vorhandenes flüchtiges Treibmittel, umfasst.

8. Verwendung nach irgendeinem der vorhergehenden Ansprüche, wobei das AASCH durch ein Verfahren hergestellt wird, das die folgenden Schritte umfasst: i) Mischen von ASCH, wasserlöslichem Calciumsalz, Aminosäure und Wasser, ii) Erwärmen der Mischung auf eine Temperatur von mindestens 65 °C und iii) Abkühlen der Mischung auf Umgebungstemperatur.

9. Verwendung nach irgendeinem der vorhergehenden Ansprüche, wobei der antimikrobielle Nutzen mindestens 5 Stunden, bevorzugt mindestens 24 Stunden anhält.

10. Verwendung nach irgendeinem der vorhergehenden Ansprüche, wobei das AASCH topisch auf die Unterarmregionen des menschlichen Körpers angewendet wird.

11. Verwendung irgendeinem der vorhergehenden Ansprüche, wobei das AASCH aus einem kosmetischen Deoroller oder einer kosmetischen Aerosolzusammensetzung aufgebracht wird.

12. Aktiviertes Aluminiumsesquichlorhydrat (AASCH) zur Verwendung als antimikrobielles Mittel gegen Staphylokokken auf der Oberfläche des menschlichen Körpers, **dadurch gekennzeichnet, dass** das AASCH ein wasserlösliches Calciumsalz, eine Aminosäure und ASCH der Formel Al₂OH_{4,4}Cl_{1,6} bis Al₂OH_{4,9}Cl_{1,1} umfasst.

13. Aktiviertes Aluminiumsesquichlorhydrat (AASCH) zur Verwendung als antimikrobielles Mittel nach Anspruch 12, **dadurch gekennzeichnet, dass** das AASCH ein wasserlösliches Calciumsalz, eine Aminosäure und ASCH der Formel Al₂OH_{4,7}Cl_{1,3} bis Al₂OH_{4,9}Cl_{1,1} umfasst.

## Revendications

1. Utilisation non thérapeutique d'un sesquichlorhydrate d'aluminium activé (AASCH) pour réduire les mauvaises odeurs dues à des staphylocoques sur la surface du corps humain, **caractérisée en ce que** l'AASCH comprend un sel de calcium soluble dans l'eau, un acide aminé et du sesquichlorhydrate d'aluminium (ASCH) de formule Al₂OH_{4,4}Cl_{1,6} à Al₂OH_{4,9}Cl_{1,1}.

2. Utilisation non thérapeutique d'un sesquichlorhydrate d'aluminium activé (AASCH) selon la revendication 1, **caractérisée en ce que** l'AASCH comprend un sel de calcium soluble dans l'eau, un acide aminé et du sesquichlorhydrate d'aluminium (ASCH) de formule Al₂OH_{4,7}Cl_{1,3} à Al₂OH_{4,9}Cl_{1,1}.

3. Utilisation selon la revendication 1 ou la revendication 2, dans laquelle le rapport molaire du sel d'aluminium basique au calcium est de 1/20 à 1/5 et le rapport du sel d'aluminium basique à l'acide aminé est de 1/10 à 1/1.

4. Utilisation selon l'une quelconque des revendications précédentes, dans laquelle l'acide aminé est la glycine et le sel de calcium soluble dans l'eau est le chlorure de calcium.

5. Utilisation selon l'une quelconque des revendications précédentes, dans laquelle le chlorure d'aluminium a une teneur en bande III, mesurée par SEC, supérieure à 30 %.

6. Utilisation selon l'une quelconque des revendications précédentes, dans laquelle l'AASCH est appliqué à partir d'une composition cosmétique choisie parmi un aérosol, un liquide, un solide mou ou un solide, de préférence à partir d'un aérosol ou d'un liquide.

7. Utilisation selon la revendication 5, dans laquelle l'AASCH représente de 5 à 50 % en poids de la composition totale à l'exclusion d'un quelconque propulseur volatil présent.

8. Utilisation selon l'une quelconque des revendications précédentes, dans laquelle l'AASCH est fabriqué par un procédé comprenant les étapes de : i) mélange d'ASCH, de sel de calcium soluble dans l'eau, d'acide aminé et d'eau, ii) chauffage du mélange à une température d'au moins 65°C, et iii) retour du mélange à la température ambiante.

9. Utilisation selon l'une quelconque des revendications précédentes, dans laquelle le bénéfice antimicrobien dure pendant au moins 5 heures, de préférence au moins 24 heures.

10. Utilisation selon l'une quelconque des revendications précédentes, dans laquelle l'AASCH est administré par voie topique aux régions axillaires du corps humain.

11. Utilisation selon l'une quelconque des revendications précédentes, dans laquelle l'AASCH est appliqué à partir d'une composition cosmétique à bille ou en aérosol.

12. Sesquichlorhydrate d'aluminium activé (AASCH) pour une utilisation en tant qu'antimicrobien contre des staphylocoques sur la surface du corps humain, **caractérisé en ce que** l'AASCH comprend un sel de calcium soluble dans l'eau, un acide aminé et un ASCH de formule Al₂OH_{4,4}Cl_{1,6} à Al₂OH_{4,9}Cl_{1,1}.

13. Sesquichlorhydrate d'aluminium activé (AASCH) pour une utilisation en tant qu'antimicrobien selon la revendication 12, **caractérisé en ce que** l'AASCH comprend un sel de calcium soluble dans l'eau, un acide aminé et un ASCH de formule Al₂OH_{4,7}Cl_{1,3} à Al₂OH_{4,9}Cl_{1,1}.
